# EUROPEAN PATENT SPECIFICATION

(11) **EP 4 236 808 B1**
(45) Date of publication and mention of the grant of the patent: **26.08.2026**
(21) Application number: 21819256.5
(22) Date of filing: 19.10.2021
(51) Int. Cl.: A61B 7/00, A61B 7/02, A61B 5/024, G01N 27/327, A61B 5/145, A61B 5/1486, A61B 5/1473

(54) **GLUCOSE BIOSENSORS COMPRISING DIRECT ELECTRON TRANSFER ENZYMES AND METHODS OF MAKING AND USING THEM**
GLUCOSEBIOSENSOREN MIT DIREKTEN ELEKTRONENTRANSFERENZYMEN UND VERFAHREN ZUR IHRER HERSTELLUNG UND VERWENDUNG
BIOCAPTEURS DE GLUCOSE COMPRENANT DES ENZYMES DE TRANSFERT D'ÉLECTRONS DIRECT ET LEURS PROCÉDÉS DE FABRICATION ET D'UTILISATION

(30) Priority: 29.10.2020 US 202017084380
(43) Date of publication of application: 06.09.2023
(73) Proprietor: Medtronic MiniMed, Inc., Northridge, CA 91325 (US)
(72) Inventor: ONG, Quyen, Arcadia, California 91007 (US)
(74) Representative: Zimmermann & Partner Patentanwälte mbB
(86) International application number: PCT/US2021/055558
(87) International publication number: WO 2022/093574

(56) References cited:
- US-A1- 2010 030 045
- US-A1- 2016 290 987

## Description

### TECHNICAL FIELD

The present invention relates to methods and materials useful for implantable medical devices, such as glucose sensors used in the management of diabetes.

### BACKGROUND OF THE INVENTION

Sensors are used to monitor a wide variety of compounds in various environments, including *in vivo* analytes. The quantitative determination of analytes in humans is of great importance in the diagnoses and maintenance of a number of pathological conditions. Illustrative analytes that are commonly monitored in a large number of individuals include glucose, lactate, cholesterol, and bilirubin. The determination of glucose concentrations in body fluids is of particular importance to diabetic individuals, individuals who must frequently check glucose levels in their body fluids to regulate the glucose intake in their diets. The results of such tests can be crucial in determining what, if any, insulin and/or other medication need to be administered.

Analyte sensors typically include components that convert interactions with analytes into detectable signals that can be correlated with the concentrations of the analyte. For example, some glucose sensors use amperometric means to monitor glucose *in vivo.* Such amperometric glucose sensors typically incorporate electrodes coated with glucose oxidase, an enzyme that catalyzes the reaction between glucose and oxygen to yield gluconic acid and hydrogen peroxide (H₂O₂). The H₂O₂ formed in this reaction alters an electrode current to form a detectable and measurable signal. Based on the signal, the concentration of glucose in the individual can then be measured.

A typical electrochemical glucose sensor works according to the following chemical reactions: $GLUCOSE + {O}_{2} {\to }^{GLUCOSE OXIDASE} GLUCONIC ACID + {H}_{2} {O}_{2}$ ${H}_{2} {O}_{2} \to {O}_{2} + 2 {H}^{+} + 2 {e}^{-}$ The glucose oxidase is used to catalyze the reaction between glucose and oxygen to yield gluconic acid and hydrogen peroxide as shown in equation 1. The H₂O₂ reacts electrochemically as shown in equation 2, and the current is measured by a potentiostat. The stoichiometry of the reaction provides challenges to developing *in vivo* sensors. In particular, for optimal glucose oxidase based sensor performance, sensor signal output should be determined only by the analyte of mterest (glucose), and not by any co-substrates (O₂) or kinetically controlled parameters such as diffusion. If oxygen and glucose are present in equimolar concentrations, then the H₂O₂ is stoichiometrically related to the amount of glucose that reacts with the glucose oxidase enzyme; and the associated current that generates the sensor signal is proportional to the amount of glucose that reacts with the glucose oxidase enzyme. If, however, there is insufficient oxygen for all of the glucose to react with the glucose oxidase enzyme, then the current will be proportional to the oxygen concentration, not the glucose concentration, a phenomenon which can compromise the accuracy of glucose sensor readings (and consequently, this phenomenon is termed the "oxygen deficit problem").

In view of issues such as the oxygen deficit problem discussed above, there is a need in the art for electrochemical sensors having architectures and materials selected to avoid the oxygen deficit problem and facilitate sensor function. Embodiments of the invention disclosed herein meet these as well as other needs.
US 2016/290987 A1 relates to a biosensor comprising an electrode for measuring the hematocrit value.

### SUMMARY OF THE INVENTION

The invention is defined in the appended claims. Conventional glucose biosensors that rely upon glucose oxidase to sense glucose exhibit certain inherent challenges, including oxygen dependency, as well as the requirement for the use of a high operating potentials in analyte sensing, which can lead to the electro-oxidation of confounding interfering species such as acetaminophen and ascorbic acid. As disclosed herein, we have developed an oxygen independent glucose biosensor based on a glucose selective direct electron transfer enzyme (cellobiose dehydrogenase) and method of fabricating such sensors. The cellobiose dehydrogenase enzyme used in these sensors eliminates the need for oxygen through a self-mediating intra-domain found on the enzyme, while allowing the sensor to operate at a significantly lower operating potential than glucose oxidase based sensors (e.g. 0 mV), thereby reducing the susceptibility of the device to interfering substances.

The disclosure provides glucose sensors having constellations of layered materials that provide the devices with enhanced functional and/or material properties, for example an ability to sense glucose in the absence of O₂. The instant disclosure further provides methods for making and using such sensors. As discussed in detail below, typical embodiments include a glucose sensing system comprising a cellobiose dehydrogenase enzyme disposed over an electrode in combination with additional selected material layers such as glucose limiting membranes comprising cellulose acetate, wherein this constellation of sensor elements is designed to facilitate continuous monitoring of glucose in diabetic patients at operating potentials that avoid certain issues with interferents such as acetaminophen.

Embodiments include, for example, an amperometric glucose sensor system comprising a first working electrode, an analyte sensing layer disposed over the first working electrode, wherein the analyte sensing layer comprises cellobiose dehydrogenase, and an analyte modulating layer disposed over the analyte sensing layer. Typically, the analyte sensing layer comprises a cellobiose dehydrogenase polypeptide in amounts from about 10mg/mL protein to about 15.5mg/mL of protein. In certain embodiments, the analyte modulating layer comprises cellulose acetate, for example cellulose acetate in amounts from about 3 wt./% to about 10 wt./%.

Typically, the amperometric glucose sensor systems of the disclosure further comprise a processor, wherein the processor performs the steps of: assessing electrochemical signal data obtained from the first working electrode; and then computing a glucose concentration based upon the electrochemical signal data obtained from the first working electrode. Optionally, glucose is sensed by application of a voltage between 0 and 200 millivolts (e.g. at a voltage less than 40, 50, 75 or 100 millivolts) to the working electrode. In certain embodiments, an electrode surface comprises ethylene glycol diglycidyl ether (EGDGE) in operable contact (e.g. via a chemical modification such as a covalent bond) with the cellobiose dehydrogenase polypeptide. Optionally, the electrode surface comprises a KETJENBLACK composition.

Embodiments also include methods of making an electrochemical glucose sensor. Typically these methods include the steps of providing a base layer, forming a conductive layer over the base layer, wherein the conductive layer includes a working electrode (e.g. one comprising carbon or gold or platinum); forming glucose sensing layer over the conductive layer, wherein the glucose sensing layer is selected to include a cellobiose dehydrogenase composition that can alter the electrical current at the working electrode in the conductive layer in the presence of glucose; and then forming an analyte modulating layer comprising cellulose acetate over the glucose sensing layer so that the electrochemical analyte sensor is made. In some embodiments of these methods, the analyte modulating layer comprises cellulose acetate in amounts from about 3 wt./% to about 10 wt./%.

Embodiments also include methods of sensing glucose within the body of an individual, the method comprising implanting an electrochemical analyte sensor disclosed herein into the individual (e.g. an individual diagnosed with diabetes); sensing an alteration in current at the working electrode in the presence of glucose; and then correlating the alteration in current with the presence of glucose, so that glucose is sensed. Typically, in these methods, glucose is sensed by application of a voltage between 0 and 200 millivolts (e.g. using a potentiostat). Typically, in these methods, glucose is sensed using a processor that performs the steps of assessing electrochemical signal data obtained from the first working electrode; and then computing a glucose concentration based upon the electrochemical signal data obtained from the first working electrode.

Other objects, features and advantages of the present disclosure will become apparent to those skilled in the art from the following detailed description. It is to be understood, however, that the detailed description and specific examples, while indicating some embodiments of the present disclosure are given by way of illustration and not limitation.

### BRIEF DESCRIPTION OF THE DRAWINGS

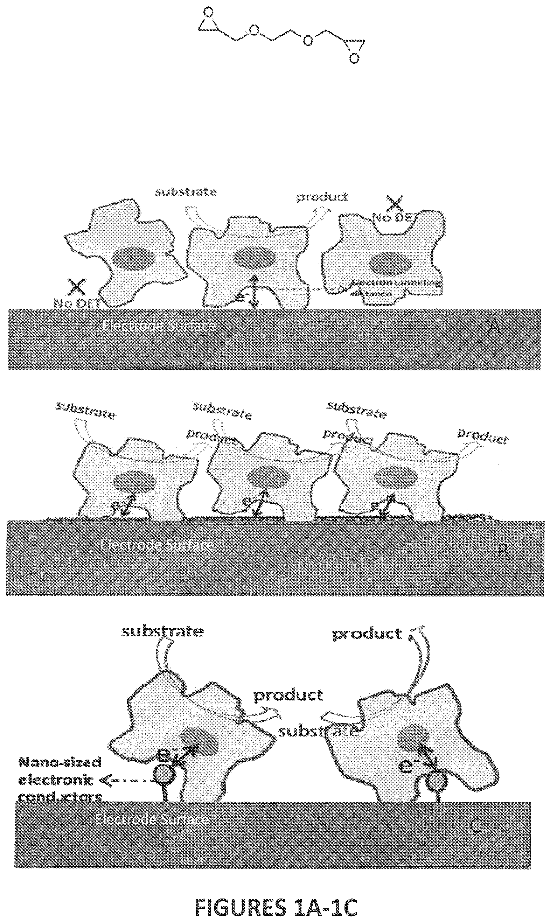

**Figures 1A-1C** provide cartoon schematics showing cellobiose dehydrogenase disposed on an electrode surface. These schematics show the importance of the orientation of the enzyme with respect to the electrode in order to facilitate direct electron transfer. Figures 1A-B highlight possible conformations in absence of any conducting polymers, whereas Figure 1C shows how presence of conducting polymer can facilitate the direct electron transfer process and hence, enzyme orientation is less critical.

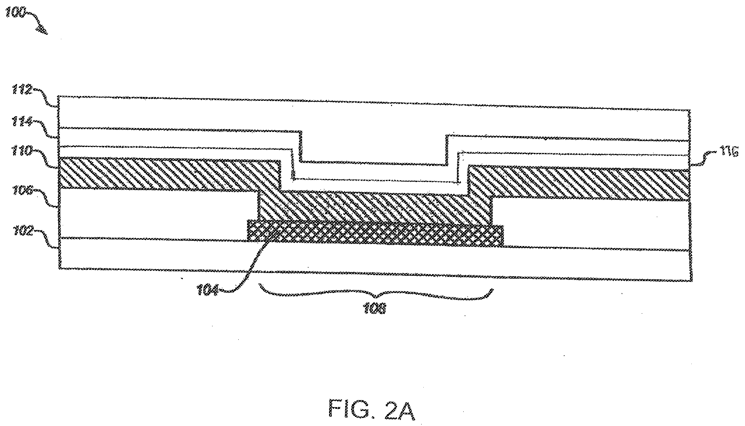

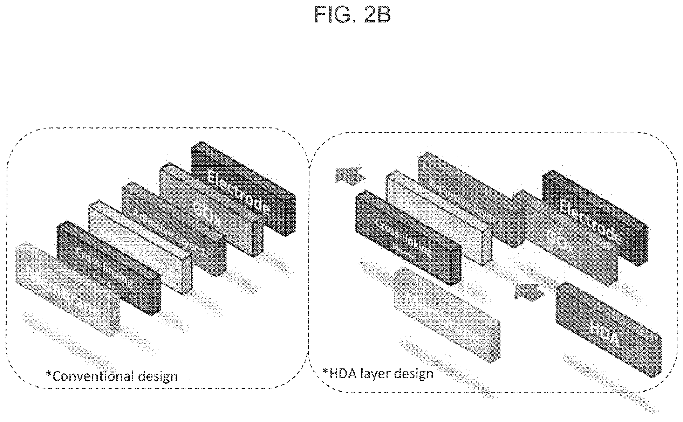

**Figures 2A-2B** provide schematics showing a conventional (PRIOR ART) sensor design comprising a first amperometric analyte sensor embodiment formed from a plurality of planar layered elements (**Figure 2A**); and a second amperometric analyte sensor embodiment having a high density amine layer (**Figure 2B**).

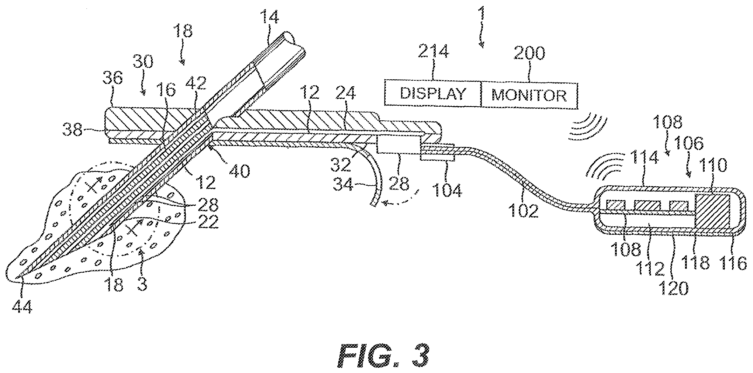

**Figure 3** provides a perspective view illustrating a subcutaneous sensor insertion set, a telemetered characteristic monitor transmitter device, and a data receiving device embodying features of the disclosure.

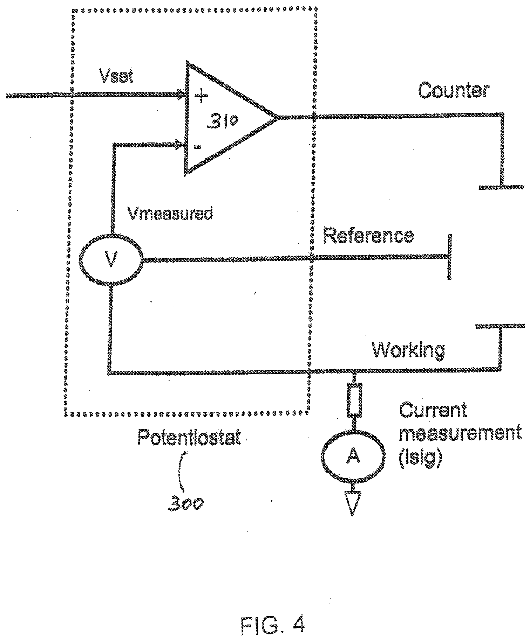

**Figure 4** shows a schematic of a potentiostat that may be used to measure current in embodiments of the present disclosure. As shown in Figure 4, a potentiostat 300 may include an op amp 310 that is connected in an electrical circuit so as to have two inputs: Vset and Vmeasured. As shown, Vmeasured is the measured value of the voltage between a reference electrode and a working electrode. Vset, on the other hand, is the optimally desired voltage between the working and reference electrodes. The effect of the op amp 310 is to control the potential on the counter electrode so as to maintain Vmeasured equal to Vset. Thus the voltage on the working electrode, which is measured with respect to the reference electrode, can be readily adjusted simply by changing Vset. This is the normal principle of operation of a potentiostat. Chemical reactions, for example the action of an enzyme captured on the working electrode and glucose in the space between the working electrode and the reference electrode, give rise to electrical currents flowing in the working electrode. These currents (isig) are the output from the potentiostat, and give a quantitative measure of the chemical reactions taking place.

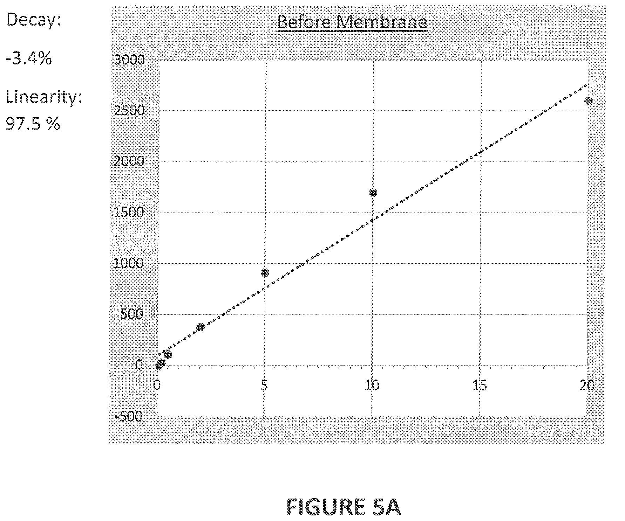

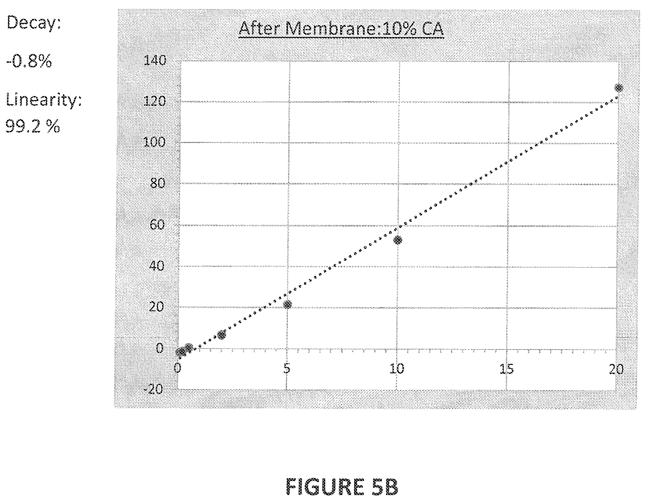

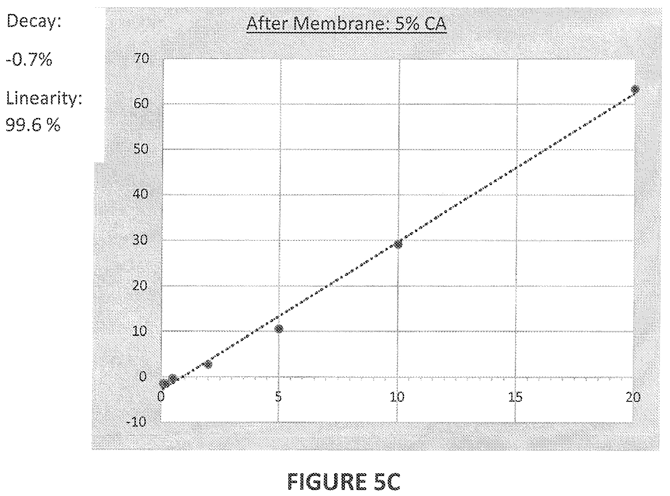

**Figures 5A-5C** provide graphed data showing sensor decay in sensors lacking a glucose limiting membrane (**Figure 5A**), sensors having a glucose limiting membrane formed from 10% cellulose acetate (**Figure 5B**) and 5% cellulose acetate (**Figure 5C**). In these graphs, the Y-axis shows current in nA, and the x-axis shows the concentration of glucose in mMol.

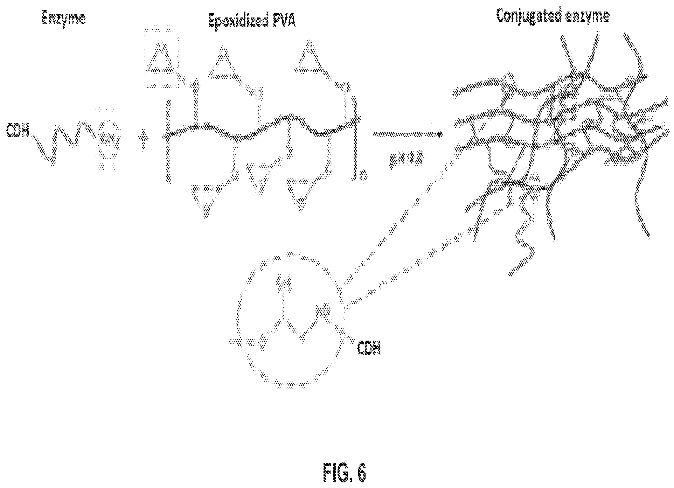

**Figure 6** provides a schematic showing the immobilization of CDH onto epoxidized PVA polymer, mimicking the EGDGE functionalization.

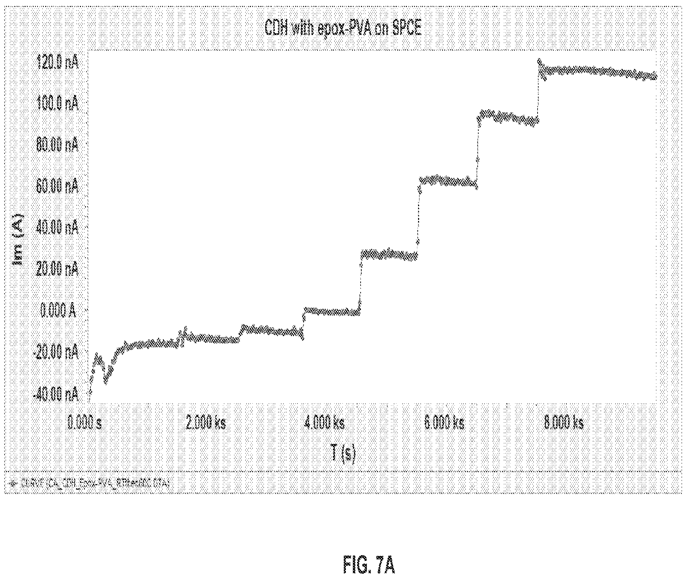

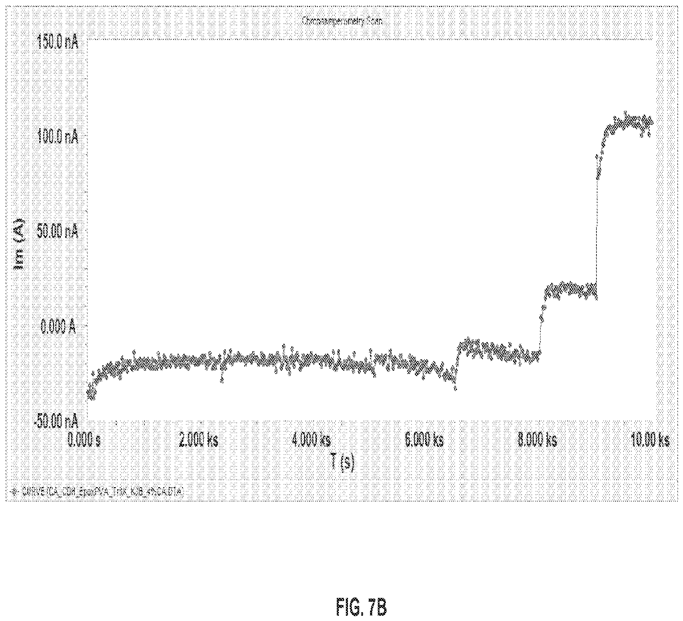

**Figures 7A-7B** provide graphed data showing glucose responses in different sensor configurations with CDH immobilized onto epoxidized PVA polymer. Fig. 7A provide graphed data showing glucose responses for CDH immobilized with epoxidized PVA in absence of a membrane. Fig. 7B provide graphed data showing glucose responses for CDH immobilized with epoxidized PVA, ketjenblack and cellulose acetate membrane (4wt/v%).

### DETAILED DESCRIPTION

Unless otherwise defined, all terms of art, notations, and other scientific terms or terminology used herein are intended to have the meanings commonly understood by those of skill in the art to which this disclosure pertains. In some cases, terms with commonly understood meanings may be defined herein for clarity and/or for ready reference, and the inclusion of such definitions herein should not necessarily be construed to represent a substantial difference over what is generally understood in the art. Many of the techniques and procedures described or referenced herein are well understood and commonly employed using conventional methodology by those skilled in the art. As appropriate, procedures involving the use of commercially available kits and reagents are generally carried out in accordance with manufacturer defined protocols and/or parameters unless otherwise noted. A number of terms are defined below.

All numbers recited in the specification and associated claims that refer to values that can be numerically characterized with a value other than a whole number (e.g. the diameter of a circular disc) are understood to be modified by the term "about". Where a range of values is provided, it is understood that each intervening value, to the tenth of the unit of the lower limit unless the context clearly dictates otherwise, between the upper and lower limit of that range and any other stated or intervening value in that stated range, is encompassed within the disclosure.

The upper and lower limits of these smaller ranges may independently be included in the smaller ranges, and are also encompassed within the disclosure, subject to any specifically excluded limit in the stated range. Where the stated range includes one or both of the limits, ranges excluding either or both of those included limits are also included in the disclosure. Publications cited herein are cited for their disclosure prior to the filing date of the present application. Nothing here is to be construed as an admission that the inventors are not entitled to antedate the publications by virtue of an earlier priority date or prior date of disclosure.

Further the actual publication dates may be different from those shown and require independent verification.

The term "analyte" as used herein is a broad term and is used in its ordinary sense, including, without limitation, to refer to a substance or chemical constituent in a fluid such as a biological fluid (for example, blood, interstitial fluid, cerebral spinal fluid, lymph fluid or urine) that can be analyzed. Analytes can include naturally occurring substances, artificial substances, metabolites, and/or reaction products. In common embodiments, the analyte is glucose. However, embodiments can be used with sensors designed for detecting a wide variety other analytes. Illustrative analytes include but are not limited to, lactate as well as salts, sugars, proteins fats, vitamins and hormones that naturally occur *in vivo* (e.g. in blood or interstitial fluids). The analyte can be naturally present in the biological fluid or endogenous; for example, a metabolic product, a hormone, an antigen, an antibody, and the like. Alternatively, the analyte can be introduced into the body or exogenous, for example, a contrast agent for imaging, a radioisotope, a chemical agent, a fluorocarbon-based synthetic blood, or a drug or pharmaceutical composition, including but not limited to insulin. The metabolic products of drugs and pharmaceutical compositions are also contemplated analytes.

The term "sensor" for example in "analyte sensor," is used in its ordinary sense, including, without limitation, means used to detect a compound such as an analyte. A "sensor system" includes, for example, elements, structures and architectures (e.g. specific 3-dimensional constellations of elements) designed to facilitate sensor use and function. Sensor systems can include, for example, compositions such as those having selected material properties, as well as electronic components such as elements and devices used in signal detection and analysis (e.g. current detectors, monitors, processors and the like).

Embodiments disclosed herein provide sensors of the type used, for example, in subcutaneous or transcutaneous monitoring of blood glucose levels in a diabetic patient. A variety of implantable, electrochemical biosensors have been developed for the treatment of diabetes and other life-threatening diseases. Many existing sensor designs use some form of immobilized enzyme to achieve their bio-specificity. Embodiments described herein can be adapted and implemented with a wide variety of known electrochemical sensors, including for example, U.S. Patent Application No. 20050115832, U.S. Pat. Nos. 6,001,067, 6,702,857, 6,212,416, 6,119,028, 6,400,974, 6,595,919, 6,141,573, 6,122,536, 6,512,939 5,605,152, 4,431,004, 4,703,756, 6,514,718, 5,985,129, 5,390,691, 5,391, 250, 5,482,473, 5,299,571, 5,568,806, 5,494,562, 6,120,676, 6,542,765, 7,033,336 as well as PCT International Publication Numbers WO 01/58348, WO 04/021877, WO 03/034902, WO 03/035117, WO 03/035891, WO 03/023388, WO 03/022128, WO 03/022352, WO 03/023708, WO 03/036255, WO03/036310 WO 08/042,625, and WO 03/074107, and European Patent Application EP 1153571.

### Illustrative Embodiments and Associated Characteristics

The disclosure has a number of embodiments. Embodiments include, for example, an amperometric glucose sensor system comprising a first working electrode (e.g. one comprising gold, one comprising platinum, one comprising a carbon paste or the like), an analyte sensing layer disposed over the first working electrode, wherein the analyte sensing layer comprises cellobiose dehydrogenase, and an analyte modulating layer disposed over the analyte sensing layer. In typical embodiments, the working electrode comprises a carbon paste electrode. The sensors disclosed herein exhibit improved longevity and accuracy as the sensor is oxygen independent, as well as being relatively interferent free.

As noted above, embodiments include sensors where the analyte sensing layer comprises cellobiose dehydrogenase. Cellobiose dehydrogenase (EC 1.1.99.18, CDH) was first discovered in 1974 in the extracellular enzyme system of Phanerochaete chrysosporium and later on in several other basidiomycetous fungi. Cloning and sequence analysis of CDHs are e.g. described in Zamocky et al. (2008) Protein Expression and Purification 59 (2): 258-265. CDH or its flavodehydrogenase domain oxidises carbohydrates like its natural substrates cellobiose and cello-oligo saccharides and others like lactose and maltose. CDHs have been discovered and modified previously to be capable of converting glucose efficiently (see, e.g. Harreither et al. (2011) Appl. Environ. Microbiol. 77:1804-1815; WO 2010/097462 A; Sygmund et al. (2009) New Biotechnology 225:115. In certain embodiments, the analyte sensing layer comprises a cellobiose dehydrogenase polypeptide in amounts from about 5mg/mL cellobiose dehydrogenase polypeptide to about 20 mg/mL of cellobiose dehydrogenase polypeptide (e.g. 10mg/mL cellobiose dehydrogenase polypeptide to about 15 mg/mL of cellobiose dehydrogenase polypeptide). Optionally, the analyte sensing layer comprises a cellobiose dehydrogenase polypeptide having at least a 90% or a 95% sequence identity to a cellobiose dehydrogenase polypeptide disclosed in Jean-Claude Sigoillot et al., ADVANCES IN BOTANICAL RESEARCH, 2012; Zamocky et al. (2008) Protein Expression and Purification 59 (2): 258-265, Harreither et al. (2011) Appl. Environ. Microbiol. 77:1804-1815; WO 2010/097462 A; Sygmund et al. (2009) New Biotechnology 225: 115, Tavahodoi et al., CemPlusChem Volume 82, Issue 4, April 2017 Pages 546-552; Scheiblbrandner et al., Bioelectrochemistry Volume 131, February 2020; WO 2010/097462, U.S. Patent Publication numbers 2015/0083611 or 2017/0247666. Such embodiments include, for instance, variant dehydrogenase polypeptides wherein one or more amino acid residues are substituted, added, or deleted.

In certain embodiments of the invention, the analyte modulating layer comprises cellulose acetate, for example cellulose acetate in amounts from 1 wt./% to 10 wt./%. Cellulose acetate materials and methods that can be adapted for use with the claimed invention are disclosed, for example, in Gunasingham et al., Biosensors Volume 4, Issue 6, 1989, Pages 349-359 and U.S. Patent Publication number 20090126570. In illustrative embodiments, one can prepare a 1-10 wt/v% solution of cellulose acetate in various solvent blends; allow this solution to stir at room temperature in capped vial for 3 hours before using; and then aspirate the solution using a dispensing tip and deposit 100uL onto center of working electrode, followed by allowing the solution to dry at room temperature for 5 mins before use.

Typically, the amperometric glucose sensor systems of the disclosure further comprise a processor, wherein the processor performs the steps of: assessing electrochemical signal data obtained from the first working carbon paste electrode (while typical embodiments use a carbon paste electrode, other electrode materials can be used in embodiments of the disclosure); and then computing a glucose concentration based upon the electrochemical signal data obtained from the first working carbon paste electrode. Optionally, glucose is sensed by application of a voltage between 0 and 200 millivolts (e.g. at a voltage less than 40, 50, 75, 100, 125, or 150 millivolts) to the working carbon paste electrode. In certain embodiments, a carbon paste electrode surface comprises ethylene glycol diglycidyl ether (EGDGE) in operable contact (e.g. via a chemical modification or functionalization) with the cellobiose dehydrogenase polypeptide. Ethylene glycol diglycidyl ether materials and methods that can be adapted for use with the claimed invention are disclosed, for example, in U.S. Patent Publication numbers 2012/0152762 and 2014/0216931. In illustrative embodiments, carbon electrode pretreatment consists of preparing a 0.1M NaOH solution; preparing a 1 wt/v% EGDGE (Ethylene glycol diglycidyl ether, Polysciences Inc., 01479-10) in 0.1M NaOH solution; immersing carbon electrodes in a 1 wt/v% EGDGE solution and leaving them in a sealed container at 60°C for 1 hour; rinsing the electrodes with deionized water and then drying them over nitrogen; followed by depositing 1uL of enzyme (e.g. previously dissolved in 0.1M PBS pH 7.4 buffer, at 15.5mg protein/mL or 10mg/mL) onto electrode; and then allowing reactions in an open vessel at 60°C for 1 hour.

Optionally, the carbon paste electrode surface comprises a IETJENBLACK composition. KETJENBLACK is a unique electro-conductive carbon black having superior performance and stability of quality. Mixed with plastic, rubber or other materials, KETJENBLACK provides the same level of electro-conductivity with a lower loading quantity as conventional carbon black. KETJENBLACK materials and methods that can be adapted for use with the claimed invention are disclosed, for example, in U.S. Patent Publication numbers 20130095384 and 2013/0058008. Embodiments utilize a screen-printed carbon working electrode (WE). The screen-printed carbon electrode is then pretreated chemically with EGDGE (or a combination of EGDGE modified carbon black) prior to deposition of the enzyme. EGDGE allows for optimal orientation of the enzyme on the electrode surface and enhances the current density. In embodiments, KETJENBLACK modified with EGDGE allows for covalent attachment of the electrode to the enzyme, and facilitates cross-linking of the enzyme to this conductive polymer (KETJENBLACK in enzyme formulation acts as conductive polymer thereby facilitating transfer of electrons from enzyme to electrode). In addition, in embodiments of the disclosure, EGDGE pretreatment of KETJENBLACK helps with enzyme orientation with respect to proximity of enzyme to KETJENBLACK (while orientation is no longer as critical on planar surface when the enzyme is surrounded by conductive polymers).

KETJENBLACK (registered trademark of Nouryon Functional Chemicals B.V. of Zutphenseweg 10 7418 AJ, Deventer , OVERIJSSEL Netherlands) is a form of very pure electro-conductive carbon black (CAS number 1333-86-4, EINECS/ELINCS No. 215-609-9) in which "crystallites come together to form primary particles which in turn adhere to form structures that then develop into agglomerates through the action of Van der Waals forces". (definition: Dictionary of Energy - Cutler J. Cleveland and Christopher Morris 2014). KETJENBLACK is typically produced in a furnace by the incomplete combustion of an oil stream. It is used as a polymer additive to impart electrical conductivity. KETJENBLACK itself is a material typically with an intrinsic resistivity of about 0.01 - 0.1 [Ω·cm]. It is available from Nouryon as pellets of around 3 mm. These pellets can be easily broken into the smaller particles that constitute them: the agglomerates, with a size of roughly 100 micrometers (µm). Agglomerates are formed by aggregates, which are roughly 100nm - 1000 nm in size, and strongly adhere to each other. The basic carbon crystallite within the aggregates is 30nm - 50nm. KETJENBLACK is available from Nouryon as either Ketjenblack EC-300J Nouryon which has a surface area of approx. 800 m2/g (BET), or as Ketjenblack EC-600JD which has a surface area of approx. 1400 m2/g (BET). For more information on commercially available KETJENBLACK, reference is made to datasheets at: https://www.nouryon.com/product/ketjenblack-ec-300j/, https://www.nouryon.com/product/ketjenblack-ec-600jd-electroconductive-carbon-black-cas-1333-86-4/, and https://www.nouryon.com/globalassers/inriver/resources/pds-ketjenblack-ec-600jd-polymer-processing-glo-en.pdf.

In the description herein references to KETJENBLACK, unless otherwise stated refer to KETJENBLACK EC-600JD available from Nouryon and referred to in its data sheet referred to above. In summary IETJENBLACK EC-600JD Ketjenblack EC-600JD is a pure electro-conductive carbon black with a surface area of approx. 1400 m2/g (BET). It has an apparent bulk density 100-120 kg/m³, appearance of black free-flowing pellets, ash content less than of equal to 0.1 %, fines less than 125 micron less than or equal to 7 %, grit content less than or equal to 30 mg/kg, iodine absorption 1000-1100 mg/g, moisture less than or equal to 0.5 %, pH 9.0-10.5, pore volume, DBP 480-510 ml/100g and volatiles less than or equal to 1.0 %.

In some embodiments, cellobiose dehydrogenase is coupled (e.g. covalently crosslinked) to a polymer such as a polyvinyl alcohol (see, e.g. Shinde et al., Biotechnology Reports 19 (2018)). Certain embodiments can use an epoxidized PVA composition to cross-link the enzyme into the PVA (see, e.g. Kazemnejadi, Milad & Eslahi, Hassan & Sardarian, Alireza; (2016) A New Approach to Cross-Linking of Polyvinyl Alcohol and Its Swelling Studies and Tao Shui, Michael Chae and David C. Bressler, Cross-Linking of Thermally Hydrolyzed Specified Risk Materials with Epoxidized Poly (Vinyl Alcohol) for Tackifier Application). The use of epoxidized PVA can create a matrix for immobilization of the cellobiose dehydrogenase enzyme to prevent desorption and hence inhibit loss of electrical communication between the enzyme and the electrode. In some embodiments, the cellobiose dehydrogenase enzyme can be coupled to the polymer in this manner in combination with an EGDGE chemical pretreatment which results in cross-linking of the enzyme to a film in order to provide additional stability to the sensor. In other embodiments, the cellobiose dehydrogenase enzyme can be coupled to an epoxidized PVA, with the use of a hydrophilic polymer support enhancing the final stability of the enzyme, with the immobilization performed at mild conditions because of the stability of the epoxy groups, the immobilization may be performed for long periods of time under a range of conditions including alkaline pH (e.g., pH 10) (aspects of such processes are shown in Figures 6 and 7). Furthermore, hydrophilic zones of the enzyme can be reacted with the epoxy groups of the support enabling the enzyme to be orientated optimally, further facilitating direct electron transfer.

The synthesis of the epoxidized PVA is reported by Bressler *et.al.* Cross-Linking of Thermally Hydrolyzed Specified Risk Materials with Epoxidized Poly (Vinyl Alcohol) for Tackifier Application, and their procedure is adapted for use with the present disclosure. Briefly, a number of PVA (different molecular weights with varying degrees of hydrolysis) can be employed (Mw 13,000-67,000 and 87-98% degree of hydrolysis, more preferably Mw 13,000-23,000 87-89% hydrolyzed). The molar ratio of hydroxyl groups to epoxy groups can also be modified (typical range 1:0.5 to 1:4 hydroxy to epoxy groups, more preferred ratio being 1:3).

In an example of the immobilization procedure, cellobiose dehydrogenase is mixed with the epoxidized PVA (2wt/v% to 15 wt/v%, more preferably at range of 5 to 10 wt/v%) at pH 7-10 (more preferably at pH range 8-9) and allowed to react for 10 minutes to 20 hours at room (more preferably at 60C for 1 hour), see figure 6. In embodiments, the cellobiose dehydrogenase can be coupled to this polymer in the presence of a conductive polymer (e.g. KETJENBLACK 0.1 to 0.3 wt/v%, various water soluble polyanilines) and deposited on a carbon electrode. In other embodiments, the same formulation can also be deposited on gold electrodes as this polymer serves the role of the EGDGE chemical pretreatment and allows the enzyme to be more optimally orientated further facilitating direct electron transfer and amplifying the current density previously observed when using EGDGE. Aspects of such processes are shown in Figures 6 and 7. It is to be understood that for those familiar with the art, modifications to the epoxy PVA support can also be made to further stabilize the enzyme and these include other heterofunctional supports prepared by modification of the epoxy groups and include various heterofunctional amino epoxy and thiol epoxy functionalization.

In certain embodiments, the glucose sensor system further comprises additional elements disclosed herein such as a counter electrode and a reference electrode; and/or one or more additional layers disposed over the analyte modulating layer selected from: a layer comprising poly-l-lysine polymers having molecular weights between 30 KDa and 300KD, or a layer comprising a polyelectrolyte. As is known in the art, polyelectrolytes are polymers whose repeating units bear an electrolyte group. In certain embodiments, this layer is a layer comprising a polycationic composition, for example one comprising a poly L lysine, a polyethleneimine (PEI), a PDDMAC = poly (diallyldimethylammonium chloride) or the like. Trehalose is another good example of a polyelectrolyte which can used in varying amounts from 0.1 to 0.3wt% in embodiments. The stabilization of enzymes and proteins using various polyelectrolytes and reagents is known in the art and described for example, in Gavalas et al., Biosensors & Bioelectronics 13 (1998) 1205-1211.

Embodiments also include methods of making an electrochemical glucose sensor. Typically these methods include the steps of providing a base layer, forming a conductive layer over the base layer, wherein the conductive layer includes a carbon working electrode; forming glucose sensing layer over the conductive layer, wherein the glucose sensing layer is selected to include a cellobiose dehydrogenase composition that can alter the electrical current at the carbon working electrode in the conductive layer in the presence of glucose; and then forming an analyte modulating layer comprising cellulose acetate over the glucose sensing layer so that the electrochemical analyte sensor is made. Typically in these methods, the glucose sensing layer comprises a cellobiose dehydrogenase polypeptide selected to have an at least a 90% identity to a cellobiose dehydrogenase disclosed in in WO 2010/097462, U.S. Patent Publication numbers 2015/0083611 or 2017/02476661, and the analyte modulating layer comprises cellulose acetate in amounts from about 3 wt./% to about 5 wt./% (e.g. about 4 wt./% to about 7 wt./%). Certain embodiments of these methods further comprise disposing an ethylene glycol diglycidyl ether (EGDGE) composition on the carbon working electrode; and/or disposing a KETJENBLACK composition on the carbon working electrode. Some embodiments of these methods comprise forming a counter electrode and/or a reference electrode on the base layer; and/or disposing one or more additional layers over the analyte modulating layer such as a layer comprising poly-l-lysine polymers having molecular weights between 30 KDa and 300KDa, a layer comprising an albumin, a layer comprising an adhesion promoting agent, or a layer comprising a cationic polyelectrolyte layer.

Embodiments also include methods of sensing glucose within the body of an individual, the method comprising implanting an electrochemical analyte sensor disclosed herein into the individual (e.g. an individual diagnosed with diabetes); sensing an alteration in current at the working electrode in the presence of glucose; and then correlating the alteration in current with the presence of glucose, so that glucose is sensed. Typically in these methods, glucose is sensed by application of a voltage between 0 and 200 millivolts, for example at a voltage less than 40, 50, 75, 100, 125, or 150 millivolts (e.g. using a potentiostat). Typically in these methods, glucose is sensed using a processor that performs the steps of assessing electrochemical signal data obtained from the first working electrode; and then computing a glucose concentration based upon the electrochemical signal data obtained from the first working electrode.

Certain embodiments are designed to include a selected constellation of elements that function together in a synergistic fashion. For example, include an amperometric glucose sensor system comprising a first working carbon paste electrode having an analyte sensing layer disposed over the first working electrode, wherein the analyte sensing layer comprises cellobiose dehydrogenase in amounts from about 10 mg/mL to about 15 mg/mL; and then an analyte modulating layer disposed over the analyte sensing layer, wherein the analyte modulating layer comprises cellulose acetate in amounts from about 3 wt./% to about 10 wt./%; wherein glucose is sensed by application of a voltage less than 40, 50, 75 or 100 millivolts to the working electrode. Typically in these embodiments, a carbon paste electrode surface comprises ethylene glycol diglycidyl ether (EGDGE) in operable contact with the cellobiose dehydrogenase polypeptide. In this context, EGDGE allows for optimal orientation of the enzyme on the electrode surface and enhances the current density. In embodiments, KETJENBLACK modified with EGDGE allows for covalent attachment of the electrode to the enzyme, and facilitates cross-linking of the enzyme to this conductive polymer (KETJENBLACK in enzyme formulation acts as conductive polymer thereby facilitating transfer of electrons from enzyme to electrode). Such embodiments have the benefit of operating at a significantly lower operating potential than glucose oxidase based sensors (for example at a voltage less than 40, 50, 75, 100, 125, or 150 millivolts), which thereby reduces the susceptibility of the device to signals from interfering substances such as acetaminophen and ascorbic acid. In this embodiment, the layer of cellulose acetate functions to inhibit sensor decay of this constellation of elements (see, e.g., data presented in FIG. 5).

In typical glucose sensor embodiments, electrochemical glucose sensors are operatively coupled to a sensor input capable of receiving signals from the electrochemical sensor; and a processor coupled to the sensor input, wherein the processor is capable of characterizing one or more signals received from the electrochemical sensor. In certain embodiments, the electrical conduit of the electrode is coupled to a potentiostat. Optionally, a pulsed voltage is used to obtain a signal from an electrode. In certain embodiments, the processor is capable of comparing a first signal received from a working electrode in response to a first working potential with a second signal received from a working electrode in response to a second working potential. Optionally, the electrode is coupled to a processor adapted to convert data obtained from observing fluctuations in electrical current from a first format into a second format. Such embodiments include, for example, processors designed to convert a sensor current Input Signal (e.g. ISIG measured in nA) to a blood glucose concentration.

In embodiments, the sensors comprise another biocompatible polymer region adapted to be implanted *in vivo and* directly contact the *in vivo* environment. In embodiments, the biocompatible region can comprise any polymer surface that contacts an *in vivo* tissue. In this way, sensors used in the systems of the invention can be used to sense a wide variety of analytes in different aqueous environments. In some embodiments, the sensor comprises a discreet probe that pierces an *in vivo* environment. In some embodiments, the electrode is coupled to a piercing member (e.g. a needle) adapted to be implanted *in vivo.* While sensor embodiments can comprise one or two piercing members, optionally such sensor apparatuses can include 3 or 4 or 5 or more piercing members that are coupled to and extend from a base element and are operatively coupled to 3 or 4 or 5 or more electrochemical sensors (e.g. microneedle arrays, embodiments of which are disclosed for example in U.S. Pat. Nos. 7,291,497 and 7,027,478, and U.S. patent Application No. 20080015494.

Embodiments include analyte sensor apparatus designed to utilize the analyte sensing layers of material disclosed herein. Such apparatuses typically include a base on which electrically conductive members are disposed and configured to form a working electrode. In some embodiments, an array of electrically conductive members is coupled to a common electrical conduit (e.g. so that the conductive members of the array are not separately wired, and are instead electrically linked as a group). Optionally, the electrical conduit is coupled to a power source adapted to sense fluctuations in electrical current of the array of the working electrode. Typically, the apparatus includes a reference electrode; and a counter electrode. Optionally one or more of these electrodes also comprises a plurality of electrically conductive members disposed on the base in an array. In some embodiments, each of the electrically conductive members of the electrode (e.g. the counter electrode) comprises an electroactive surface adapted to sense fluctuations in electrical current at the electroactive surface; and the group of electrically conductive members are coupled to a power source (e.g. a potentiostat or the like).

In some embodiments, the apparatus comprises a plurality of working electrodes, counter electrodes and reference electrodes clustered together in units consisting essentially of one working electrode, one counter electrode and one reference electrode; and the clustered units are longitudinally distributed on the base layer in a repeating pattern of units. In some sensor embodiments, the distributed electrodes are organized/disposed within a flex-circuit assembly (i.e. a circuitry assembly that utilizes flexible rather than rigid materials). Such flex-circuit assembly embodiments provide an interconnected assembly of elements (e.g. electrodes, electrical conduits, contact pads and the like) configured to facilitate wearer comfort (for example by reducing pad stiffness and wearer discomfort).

In embodiments, an analyte sensing layer is disposed over electrically conductive members, and includes an agent that is selected for its ability to detectably alter the electrical current at the working electrode in the presence of an analyte. In the working embodiments of the invention that are disclosed herein, the agent is cellobiose dehydrogenase, a protein that undergoes a chemical reaction in the presence of glucose that results in an alteration in the electrical current at the working electrode. These working embodiments further include an analyte modulating layer disposed over the analyte sensing layer, wherein the analyte modulating layer modulates the diffusion of glucose as it migrates from an *in vivo* environment to the analyte sensing layer.

In embodiments, a glucose limiting membrane can be formed from known compositions designed for this purpose such as those disclosed in U.S. Patent Publication 2017/0347933. According to the invention, the use of cellulose acetate as the glucose limiting membrane allows for increased operational stability of the sensors (see, FIG. 5). According to the invention, the analyte modulating layer comprises cellulose acetate, which is observed to inhibit sensor decay more efficiently than analyte modulating layers formed from other agents such as Nafion, chitosan or polycarbonate-urethane (although these agents can also be used in analyte modulating layers of the invention). In certain embodiments, the analyte modulating layer comprises a hydrophilic comb-copolymer having a central chain and a plurality of side chains coupled to the central chain, wherein at least one side chain comprises a silicone moiety. In certain embodiments, the analyte modulating layer comprises a blended mixture of: a linear polyurethane/polyurea polymer, and a branched acrylate polymer; and the linear polyurethane/polyurea polymer and the branched acrylate polymer are blended at a ratio of between 1:1 and 1:20 (e.g. 1:2) by weight %. Typically, this analyte modulating layer composition comprises a first polymer formed from a mixture comprising a diisocyanate; at least one hydrophilic diol or hydrophilic diamine; and a siloxane; that is blended with a second polymer formed from a mixture comprising: a 2-(dimethylamino)ethyl methacrylate; a methyl methacrylate; a polydimethyl siloxane monomethacryloxypropyl; a poly(ethylene oxide) methyl ether methacrylate; and a 2-hydroxyethyl methacrylate. Additional material layers can be included in such apparatuses. For example, in some embodiments, the apparatus comprises an adhesion promoting layer disposed between the analyte sensing layer and the analyte modulating layer.

One sensor embodiment shown in Figure 2A is an amperometric sensor 100 having a plurality of layered elements. Embodiments disclosed herein can have less layers than shown in Figure 2A (e.g. no protein or adhesion promoting layers) or additional layers not shown (e.g. a polyelectrolyte layer). In Figure 2A, the embodiment includes a base layer 102, a conductive layer 104 (e.g. one comprising the plurality of electrically conductive members) which is disposed on and/or combined with the base layer 102. Typically, the conductive layer 104 comprises one or more electrodes. An analyte sensing layer 110 (typically comprising an enzyme such as cellobiose dehydrogenase) can be disposed on one or more of the exposed electrodes of the conductive layer 104. Optionally, a protein layer 116 can be disposed upon the analyte sensing layer 110. An analyte modulating layer 112 can be disposed above the analyte sensing layer 110 to regulate analyte (e.g. glucose) access with the analyte sensing layer 110. Optionally, an adhesion promoter layer 114 is disposed between layers such as the analyte modulating layer 112 and the analyte sensing layer 110 as shown in FIG. 2A in order to facilitate their contact and/or adhesion. This embodiment also comprises a cover layer 106 such as a polymer surface coating disclosed herein can be disposed on portions of the sensor 100. Apertures 108 can be formed in one or more layers of such sensors. Amperometric glucose sensors having this type of design are disclosed, for example, in U.S. Patent Application Publication Nos. 20070227907, 20100025238, 20110319734 and 20110152654.

Embodiments also provide articles of manufacture and kits for observing a concentration of an analyte. In an illustrative embodiment, the kit includes a sensor comprising a glucose sensing and/or glucose limiting membrane as discussed herein. In typical embodiments, the sensors are disposed in the kit within a sealed sterile dry package. Optionally the kit comprises an insertion device that facilitates insertion of the sensor. The kit and/or sensor set typically comprises a container, a label and an analyte sensor as described above. Suitable containers include, for example, an easy to open package made from a material such as a metal foil, bottles, vials, syringes, and test tubes. The containers may be formed from a variety of materials such as metals (e.g. foils) paper products, glass or plastic. The label on, or associated with, the container indicates that the sensor is used for assaying the analyte of choice. The kit and/or sensor set may include other materials desirable from a commercial and user standpoint, including buffers, diluents, filters, needles, syringes, and package inserts with instructions for use.

Specific aspects of embodiments are discussed in detail in the following sections.

### Typical Elements, Configurations and Analyte Sensor Embodiments

### A. Typical Elements Found in of Embodiments

Figures 2A and 2B and 3 provide illustrations of various sensor and sensor system embodiments.

Figure 2A illustrates a cross-section of a typical sensor embodiment 100. This sensor embodiment is formed from a plurality of components that are typically in the form of layers of various conductive and non-conductive constituents disposed on each other according to art accepted methods and/or the specific methods disclosed herein. The components of the sensor are typically characterized herein as layers because, for example, it allows for a facile characterization of the sensor structure shown in Figure 2A. Artisans will understand however, that in certain embodiments, the sensor constituents are combined such that multiple constituents form one or more heterogeneous layers. In this context, those of skill in the art understand that the ordering of the layered constituents can be altered in various embodiments.

The embodiment shown in Figure 2A includes a base layer 102 to support the sensor 100. The base layer 102 can be made of a material such as a metal and/or a ceramic, which may be self-supporting or further supported by another material as is known in the art. Embodiments include a conductive layer 104 which is disposed on and/or combined with the base layer 102. Typically, the conductive layer 104 comprises one or more electrically conductive elements that function as electrodes. An operating sensor 100 typically includes a plurality of electrodes such as a working electrode, a counter electrode and a reference electrode. Other embodiments may also include a plurality of working and/or counter and/or reference electrodes and/or one or more electrodes that performs multiple functions, for example one that functions as both as a reference and a counter electrode.

As discussed in detail below, the base layer 102 and/or conductive layer 104 can be generated using many known techniques and materials. In certain embodiments, the electrical circuit of the sensor is defined by etching the disposed conductive layer 104 into a desired pattern of conductive paths. A typical electrical circuit for the sensor 100 comprises two or more adjacent conductive paths with regions at a proximal end to form contact pads and regions at a distal end to form sensor electrodes. An electrically insulating cover layer 106 such as a polymer coating can be disposed on portions of the sensor 100. Acceptable polymer coatings for use as the insulating protective cover layer 106 can include, but are not limited to polymers having the constellation of features disclosed herein, non-toxic biocompatible polymers such as silicone compounds, polyimides, biocompatible solder masks, epoxy acrylate copolymers, or the like. In the sensors of the present disclosure, one or more exposed regions or apertures 108 can be made through the cover layer 106 to open the conductive layer 104 to the external environment and to, for example, allow an analyte such as glucose to permeate the layers of the sensor and be sensed by the sensing elements. Apertures 108 can be formed by a number of techniques, including laser ablation, tape masking, chemical milling or etching or photolithographic development or the like. In certain embodiments, during manufacture, a secondary photoresist can also be applied to the protective layer 106 to define the regions of the protective layer to be removed to form the aperture(s) 108. The exposed electrodes and/or contact pads can also undergo secondary processing (e.g. through the apertures 108), such as additional plating processing, to prepare the surfaces and/or strengthen the conductive regions.

In the sensor configuration shown in Figure 2A, an analyte sensing layer 110 is disposed on one or more of the exposed electrodes of the conductive layer 104. Typically, the analyte sensing layer 110 is an enzyme layer. Most typically, the analyte sensing layer 110 comprises the enzyme cellobiose dehydrogenase. Optionally the enzyme in the analyte sensing layer is combined with a second carrier protein such as human serum albumin, bovine serum albumin or the like. In an illustrative embodiment, an enzyme such as cellobiose dehydrogenase in the analyte sensing layer 110 reacts with glucose and modulate current at an electrode.

In embodiments, the analyte sensing layer 110 can be applied over portions of the conductive layer or over the entire region of the conductive layer. Typically, the analyte sensing layer 110 is disposed on the working electrode which can be the anode or the cathode. Optionally, the analyte sensing layer 110 is also disposed on a counter and/or reference electrode. Methods for generating a thin analyte sensing layer 110 include brushing the layer onto a substrate (e.g. the reactive surface of a platinum black electrode), as well as spin coating processes, dip and dry processes, low shear spraying processes, ink-jet printing processes, silk screen processes and the like. In certain embodiments, brushing is used to: (1) allow for a precise localization of the layer; and (2) push the layer deep into the architecture of the reactive surface of an electrode (e.g. platinum black produced by an electrodeposition process).

Typically, the analyte sensing layer 110 is coated and or disposed next to one or more additional layers. Optionally, the one or more additional layers includes a protein layer 116 disposed upon the analyte sensing layer 110. Typically, the protein layer 116 comprises a protein such as human serum albumin, bovine serum albumin or the like. Typically, the protein layer 116 comprises human serum albumin. In some embodiments, an additional layer includes an analyte modulating layer 112 that is disposed above the analyte sensing layer 110 to regulate analyte contact with the analyte sensing layer 110. For example, the analyte modulating membrane layer 112 can comprise a glucose limiting membrane, which regulates the amount of glucose that contacts an enzyme such as cellobiose dehydrogenase that is present in the analyte sensing layer. Such glucose limiting membranes can be made from a wide variety of materials known to be suitable for such purposes, e.g., silicone compounds such as polydimethyl siloxanes, polyurethanes, polyurea cellulose acetates, Nafion, polyester sulfonic acids (e.g. Kodak AQ), hydrogels or any other suitable hydrophilic membranes known to those skilled in the art.

### B. Typical Analyte Sensor Constituents Used in Embodiments

The following disclosure provides examples of typical elements/constituents used in sensor embodiments of the disclosure. While these elements can be described as discreet units (e.g. layers), those of skill in the art understand that sensors can be designed to contain elements having a combination of some or all of the material properties and/or functions of the elements/constituents discussed below (e.g. an element that serves both as a supporting base constituent and/or a conductive constituent and/or a matrix for the analyte sensing constituent and which further functions as an electrode in the sensor). Those in the art understand that these thin film analyte sensors can be adapted for use in a number of sensor systems such as those described below.

### Base Constituent

Sensors typically include a base constituent (see, e.g. element 102 in Figure 2A). The term "base constituent" is used herein according to art accepted terminology and refers to the constituent in the apparatus that typically provides a supporting matrix for the plurality of constituents that are stacked on top of one another and comprise the functioning sensor. In one form, the base constituent comprises a thin film sheet of insulative (e.g. electrically insulative and/or water impermeable) material. This base constituent can be made of a wide variety of materials having desirable qualities such as dielectric properties, water impermeability and hermeticity. Some materials include metallic, and/or ceramic and/or polymeric substrates or the like. In some sensor embodiments, the electrode(s) on the base are organized/disposed within a flex-circuit assembly (i.e. a circuitry assembly that utilizes flexible rather than rigid materials).

### Conductive Constituent

The electrochemical sensors typically include a conductive constituent disposed upon the base constituent that includes at least one electrode for contacting an analyte or its byproduct (e.g. glucose) to be assayed (see, e.g. element 104 in Figure 2A). The term "conductive constituent" is used herein according to art accepted terminology. An illustrative example of this is a conductive constituent that forms a working electrode that can measure an increase or decrease in current in response to exposure to a stimuli such as the change in the concentration of an analyte or its byproduct as compared to a reference electrode that does not experience the change in the concentration of the analyte when the analyte interacts with a composition (e.g. the enzyme cellobiose dehydrogenase) present in analyte sensing constituent 110. Such electrodes include carbon-paste electrodes (CPE), electrodes that are typically made from a mixture of conducting graphite powder and a pasting liquid (e.g. a screen-printed carbon electrode).

In addition to the working electrode, the analyte sensors typically include a reference electrode or a combined reference and counter electrode (also termed a quasi-reference electrode or a counter/reference electrode). If the sensor does not have a counter/reference electrode then it may include a separate counter electrode, which may be made from the same or different materials as the working electrode. Typical sensors have one or more working electrodes and one or more counter, reference, and/or counter/reference electrodes. One embodiment of the sensor has two, three or four or more working electrodes. These working electrodes in the sensor may be integrally connected or they may be kept separate. Optionally, the electrodes can be disposed on a single surface or side of the sensor structure. Alternatively, the electrodes can be disposed on a multiple surfaces or sides of the sensor structure (and can for example be connected by vias through the sensor material(s) to the surfaces on which the electrodes are disposed). In certain embodiments, the reactive surfaces of the electrodes are of different relative areas/sizes, for example a 1X reference electrode, a 2.6X working electrode and a 3.6X counter electrode.

### Analyte Sensing Constituent

The electrochemical sensors include an analyte sensing constituent disposed on the electrodes of the sensor (see, e.g. element 110 in Figure 2A). The term "analyte sensing constituent" is used herein according to art accepted terminology and refers to a constituent comprising a material that is capable of recognizing or reacting with an analyte whose presence is to be detected by the analyte sensor apparatus. Typically, this material in the analyte sensing constituent produces a detectable signal after interacting with the analyte to be sensed, typically via the electrodes of the conductive constituent. In this regard, the analyte sensing constituent and the electrodes of the conductive constituent work in combination to produce the electrical signal that is read by an apparatus associated with the analyte sensor. Typically, the analyte sensing constituent comprises an oxidoreductase enzyme capable of reacting with and/or producing a molecule whose change in concentration can be measured by measuring the change in the current at an electrode of the conductive constituent, for example the enzyme cellobiose dehydrogenase. The analyte sensing constituent can coat all or a portion of the various electrodes of the sensor. In this context, the analyte sensing constituent may coat the electrodes to an equivalent degree. Alternatively, the analyte sensing constituent may coat different electrodes to different degrees, with for example the coated surface of the working electrode being larger than the coated surface of the counter and/or reference electrode.

Some sensor embodiments of this element utilize an enzyme (e.g. cellobiose dehydrogenase) that optionally has been combined with a second protein (e.g. albumin) in a fixed ratio (e.g. one that is typically optimized for cellobiose dehydrogenase stabilizing properties) and then applied on the surface of an electrode to form a thin enzyme constituent. In a typical embodiment, the analyte sensing constituent comprises a cellobiose dehydrogenase and HSA mixture. In a typical embodiment of an analyte sensing constituent having cellobiose dehydrogenase, the cellobiose dehydrogenase reacts with glucose present in the sensing environment (e.g. the body of a mammal).

As noted above, the enzyme and the second protein (e.g. an albumin) can be treated to form a crosslinked matrix (e.g. by adding a cross-linking agent to the protein mixture). As is known in the art, crosslinking conditions may be manipulated to modulate factors such as the retained biological activity of the enzyme, its mechanical and/or operational stability. Illustrative crosslinking procedures are described in U.S. patent application Ser. No. 10/335,506 and PCT publication WO 03/035891. For example, an amine cross-linking reagent, such as, but not limited to, glutaraldehyde, can be added to the protein mixture (however in certain embodiments disclosed herein, glutaraldehyde is excluded because the addition of a cross-linking reagent to the protein mixture creates a less active protein paste).

Alternative embodiments of analyte sensing constituents are not formed using glutaraldehyde, and are instead formed to include entrapped and/or crosslinked polypeptides such as cellobiose dehydrogenase crosslinked to polyvinyl alcohol (PVA, see, e.g. CAS number 9002-89-5) polymers. As is known in the art, polyvinyl alcohol reacts with aldehydes to form water insoluble polyacetals. In a pure PVA medium having a pH around 5.0, polymer reaction with dialdehydes is expected to form an acetal cross-linked structure. In certain embodiments, such crosslinking reactions can be performed using a chemical vapor deposition (CVD) process. Due to the acidity of the PVA polymer solution, crosslinking reactions in CVD systems are simple and routine. Moreover, acidic conditions can be created by introducing compounds such as acetic acid into glutaraldehyde solutions, so a CVD system can provide an acid vapor condition. In addition, the pH of the polymer medium can be adjusted by adding acidic compounds such as citric acid, polymer additives such as polylysine, HBr and the like.

Embodiments of the analyte sensing constituents include compositions having properties that make them particularly well suited for use in ambulatory glucose sensors of the type worn by diabetic individuals. Such embodiments include one or more layered elements (e.g. cellobiose dehydrogenase) coupled to or otherwise entrapped within a polymer matrix. Optionally the polymer matrix comprises PVA-SbQ. PVA-SbQ compositions for use in layered analyte sensor structures can comprise between 1 mol% and 12.5 mol% SbQ. In certain embodiments of the invention that are adapted or use in glucose sensors, the constituents in this layer are selected so that the molecular weight of the polyvinyl alcohol is between 30 kilodaltons and 150 kilodaltons and the SbQ in the polyvinyl alcohol is present in an amount between 1 mol% and 4 mol%. In some embodiments the analyte sensing layer is formed to comprise from 5% to 12% PVA by weight.

Embodiments of the analyte sensing constituents include analyte sensing layers selected for their ability to provide desirable characteristics for implantable sensors. In certain embodiments, an amount or ratio of PVA within the composition is used to modulate the water adsorption of the composition, the crosslinking density of the composition etc. Such formulations can readily be evaluated for their effects on phenomena such as H₂O adsorption, sensor isig drift and *in vivo* start up profiles. Sufficient H₂O adsorption can help to maintain a normal chemical and electrochemical reaction within amperometric analyte sensors. Consequently, it is desirable to form such sensors from compositions having an appropriate hydrophilic chemistry. In this context, the PVA-cellobiose dehydrogenase compositions disclosed herein can be used to create electrolyte hydrogels that are useful in internal coating/membrane layers and can also be coated on top of an analyte modulating layer (e.g. a glucose limiting membrane or "GLM") in order to improve the biocompatibility and hydrophilicity of the GLM layer.

As noted above, in some embodiments, the analyte sensing constituent includes an agent (e.g. cellobiose dehydrogenase) capable of producing a signal that can be sensed by the electrically conductive elements. However, other useful analyte sensing constituents can be formed from any composition that is capable of producing a detectable signal that can be sensed by the electrically conductive elements after interacting with a target analyte whose presence is to be detected. A variety of other enzymes known in the art can produce and/or utilize compounds whose modulation can be detected by electrically conductive elements such as the electrodes that are incorporated into the sensor designs described herein. Such enzymes include for example, enzymes specifically described in Table 1, pages 15-29 and/or Table 18, pages 111-112 of Protein Immobilization: Fundamentals and Applications (Bioprocess Technology, Vol 14) by Richard F. Taylor (Editor) Publisher: Marcel Dekker; Jan. 7, 1991).

### Protein Constituent

The electrochemical sensors optionally include a protein constituent disposed between the analyte sensing constituent and the analyte modulating constituent (see, e.g. element 116 in Figure 2A). The term "protein constituent" is used herein according to art accepted terminology and refers to constituent containing a carrier protein or the like that is selected for compatibility with the analyte sensing constituent and/or the analyte modulating constituent. In typical embodiments, the protein constituent comprises an albumin such as human serum albumin. The HSA concentration may vary between about 0.5%-30% (w/v). Typically the HSA concentration is about 1-10% w/v, and most typically is about 5% w/v. In alternative embodiments, collagen or BSA or other structural proteins used in these contexts can be used instead of or in addition to HSA. This constituent is typically crosslinked on the analyte sensing constituent according to art accepted protocols.

### Adhesion Promoting Constituent

The electrochemical sensors can include one or more adhesion promoting (AP) constituents (see, e.g. element 114 in Figure 2A). The term "adhesion promoting constituent" is used herein according to art accepted terminology and refers to a constituent that includes materials selected for their ability to promote adhesion between adjoining constituents in the sensor. Typically, the adhesion promoting constituent is disposed between the analyte sensing constituent and the analyte modulating constituent. Typically, the adhesion promoting constituent is disposed between the optional protein constituent and the analyte modulating constituent. The adhesion promoter constituent can be made from any one of a wide variety of materials known in the art to facilitate the bonding between such constituents and can be applied by any one of a wide variety of methods known in the art. Typically, the adhesion promoter constituent comprises a silane compound such as γ-aminopropyltrimethoxysilane.

### High-density Amine Constituent

The electrochemical sensors can include one or more high-density amine constituent layers (see, e.g. element 500 in FIG. 2B) that provide the sensors with a number of beneficial functions. Such layers can optimize sensor function, for example by acting as an adhesion promoting constituent for layers adjacent to the HDA layer, by decreasing fluctuations that can occur in glucose sensors by improving sensor initialization profiles and the like. Typically, the high-density amine constituent is disposed between and in direct contact with the analyte sensing constituent and the analyte modulating constituent. In typical embodiments, the high-density amine layer 500 comprises poly-l-lysine having molecular weights between 30 KDa and 300KDa (e.g. between 150 KDa and 300KDa). The concentrations of poly-l-lysine in such high-density amine layers 500 is typically from 0.1 weight-to-weight percent to 0.5 weight-to-weight percent and the high-density amine layer 500 is from 0.1 to 0.4 microns thick.

### Analyte Modulating Constituent

The electrochemical sensors include an analyte modulating constituent disposed on the sensor (see, e.g. element 112 in Figure 2A). The term "analyte modulating constituent" is used herein according to art accepted terminology and refers to a constituent that typically forms a membrane on the sensor that operates to modulate the diffusion of one or more analytes, such as glucose, through the constituent. In certain embodiments, the analyte modulating constituent is an analyte-limiting membrane which operates to prevent or restrict the diffusion of one or more analytes, such as glucose, through the constituents. In other embodiments, the analyte-modulating constituent operates to facilitate the diffusion of one or more analytes, through the constituents. Optionally such analyte modulating constituents can be formed to prevent or restrict the diffusion of one type of molecule through the constituent (e.g. glucose), while at the same time allowing or even facilitating the diffusion of other types of molecules through the constituent (e.g. O₂).

### Cover Constituent

The electrochemical sensors can include one or more cover constituents which are typically electrically insulating protective constituents (see, e.g. element 106 in Figure 2A). Typically, such cover constituents can be in the form of a coating, sheath or tube and are disposed on at least a portion of the analyte modulating constituent. Typically such features comprise a polymer comprising a surface having the constellation of features disclosed herein that function to modulate immune response. Acceptable polymer coatings for use as the insulating protective cover constituent can include, but are not limited to, non-toxic biocompatible polymers such as silicone compounds, polyimides, biocompatible solder masks, epoxy acrylate copolymers, or the like. Further, these coatings can be photo-imageable to facilitate photolithographic forming of apertures through to the conductive constituent. A typical cover constituent comprises spun on silicone. As is known in the art, this constituent can be a commercially available RTV (room temperature vulcanized) silicone composition. A typical chemistry in this context is polydimethyl siloxane (acetoxy based).

### C. Typical Analyte Sensor System Embodiments

Embodiments of the sensor elements and sensors can be operatively coupled to a variety of other system elements typically used with analyte sensors (e.g. structural elements such as piercing members, insertion sets and the like as well as electronic components such as processors, monitors, medication infusion pumps and the like), for example to adapt them for use in various contexts (e.g. implantation within a mammal). One embodiment includes a method of monitoring a physiological characteristic of a user using an embodiment that includes an input element capable of receiving a signal from a sensor that is based on a sensed physiological characteristic value of the user, and a processor for analyzing the received signal. In typical embodiments, the processor determines a dynamic behavior of the physiological characteristic value and provides an observable indicator based upon the dynamic behavior of the physiological characteristic value so determined. In some embodiments, the physiological characteristic value is a measure of the concentration of blood glucose in the user. In other embodiments, the process of analyzing the received signal and determining a dynamic behavior includes repeatedly measuring the physiological characteristic value to obtain a series of physiological characteristic values in order to, for example, incorporate comparative redundancies into a sensor apparatus in a manner designed to provide confirmatory information on sensor function, analyte concentration measurements, the presence of interferences and the like.

Figure 4 shows a schematic of a potentiostat that may be used to measure current in embodiments. As shown in Figure 4, a potentiostat 300 may include an op amp 310 that is connected in an electrical circuit so as to have two inputs: Vset and Vmeasured. As shown, Vmeasured is the measured value of the voltage between a reference electrode and a working electrode. Vset, on the other hand, is the optimally desired voltage across the working and reference electrodes. The current between the counter and reference electrode is measured, creating a current measurement (isig) that is output from the potentiostat.

Embodiments include devices which process display data from measurements of a sensed physiological characteristic (e.g. blood glucose concentrations) in a manner and format tailored to allow a user of the device to easily monitor and, if necessary, modulate the physiological status of that characteristic (e.g. modulation of blood glucose concentrations via insulin administration). An illustrative embodiment is a device comprising a sensor input capable of receiving a signal from a sensor, the signal being based on a sensed physiological characteristic value of a user; a memory for storing a plurality of measurements of the sensed physiological characteristic value of the user from the received signal from the sensor; and a display for presenting a text and/or graphical representation of the plurality of measurements of the sensed physiological characteristic value (e.g. text, a line graph or the like, a bar graph or the like, a grid pattern or the like or a combination thereof). Typically, the graphical representation displays real time measurements of the sensed physiological characteristic value. Such devices can be used in a variety of contexts, for example in combination with other medical apparatuses. In some embodiments, the device is used in combination with at least one other medical device (e.g. a glucose sensor).

An illustrative system embodiment consists of a glucose sensor, a transmitter and pump receiver and a glucose meter. In this system, radio signals from the transmitter can be sent to the pump receiver every 5 minutes to provide providing real-time sensor glucose (SG) values. Values/graphs are displayed on a monitor of the pump receiver so that a user can self monitor blood glucose and deliver insulin using their own insulin pump. Typically, an embodiment of device disclosed herein communicates with a second medical device via a wired or wireless connection. Wireless communication can include for example the reception of emitted radiation signals as occurs with the transmission of signals via RF telemetry, infrared transmissions, optical transmission, sonic and ultrasonic transmissions and the like. Optionally, the device is an integral part of a medication infusion pump (e.g. an insulin pump). Typically, in such devices, the physiological characteristic values include a plurality of measurements of blood glucose.

Figure 3 provides a perspective view of one generalized embodiment of subcutaneous sensor insertion system and a block diagram of a sensor electronics device according to one illustrative embodiment. Additional elements typically used with such sensor system embodiments are disclosed for example in U.S. Patent Application No. 2007/0163894. Figure 3 provides a perspective view of a telemetered characteristic monitor system 1, including a subcutaneous sensor set 10 provided for subcutaneous placement of an active portion of a flexible sensor 12, or the like, at a selected site in the body of a user. The subcutaneous or percutaneous portion of the sensor set 10 includes a hollow, slotted insertion needle 14 having a sharpened tip 44, and a cannula 16. Inside the cannula 16 is a sensing portion 18 of the sensor 12 to expose one or more sensor electrodes 20 to the user's bodily fluids through a window 22 formed in the cannula 16. The sensing portion 18 is joined to a connection portion 24 that terminates in conductive contact pads, or the like, which are also exposed through one of the insulative layers. The connection portion 24 and the contact pads are generally adapted for a direct wired electrical connection to a suitable monitor 200 coupled to a display 214 for monitoring a user's condition in response to signals derived from the sensor electrodes 20. The connection portion 24 may be conveniently connected electrically to the monitor 200 or a characteristic monitor transmitter 100 by a connector block 28 (or the like).

As shown in Figure 3, in accordance with embodiments, subcutaneous sensor set 10 may be configured or formed to work with either a wired or a wireless characteristic monitor system. The proximal part of the sensor 12 is mounted in a mounting base 30 adapted for placement onto the skin of a user. The mounting base 30 can be a pad having an underside surface coated with a suitable pressure sensitive adhesive layer 32, with a peel-off paper strip 34 normally provided to cover and protect the adhesive layer 32, until the sensor set 10 is ready for use. The mounting base 30 includes upper and lower layers 36 and 38, with the connection portion 24 of the flexible sensor 12 being sandwiched between the layers 36 and 38. The connection portion 24 has a forward section joined to the active sensing portion 18 of the sensor 12, which is folded angularly to extend downwardly through a bore 40 formed in the lower base layer 38. Optionally, the adhesive layer 32 (or another portion of the apparatus in contact with in vivo tissue) includes an anti-inflammatory agent to reduce an inflammatory response and/or anti-bacterial agent to reduce the chance of infection. The insertion needle 14 is adapted for slide-fit reception through a needle port 42 formed in the upper base layer 36 and through the lower bore 40 in the lower base layer 38. After insertion, the insertion needle 14 is withdrawn to leave the cannula 16 with the sensing portion 18 and the sensor electrodes 20 in place at the selected insertion site. In this embodiment, the telemetered characteristic monitor transmitter 100 is coupled to a sensor set 10 by a cable 102 through a connector 104 that is electrically coupled to the connector block 28 of the connector portion 24 of the sensor set 10.

In the embodiment shown in Figure 3, the telemetered characteristic monitor 100 includes a housing 106 that supports a printed circuit board 108, batteries 110, antenna 112, and the cable 102 with the connector 104. In some embodiments, the housing 106 is formed from an upper case 114 and a lower case 116 that are sealed with an ultrasonic weld to form a waterproof (or resistant) seal to permit cleaning by immersion (or swabbing) with water, cleaners, alcohol or the like. In some embodiments, the upper and lower case 114 and 116 are formed from a medical grade plastic. However, in alternative embodiments, the upper case 114 and lower case 116 may be connected together by other methods, such as snap fits, sealing rings, RTV (silicone sealant) and bonded together, or the like, or formed from other materials, such as metal, composites, ceramics, or the like. In other embodiments, the separate case can be eliminated and the assembly is simply potted in epoxy or other moldable materials that is compatible with the electronics and reasonably moisture resistant. As shown, the lower case 116 may have an underside surface coated with a suitable pressure sensitive adhesive layer 118, with a peel-off paper strip 120 normally provided to cover and protect the adhesive layer 118, until the sensor set telemetered characteristic monitor transmitter 100 is ready for use.

In the illustrative embodiment shown in Figure 3, the subcutaneous sensor set 10 facilitates accurate placement of a flexible thin film electrochemical sensor 12 of the type used for monitoring specific blood parameters representative of a user's condition. The sensor 12 monitors glucose levels in the body, and may be used in conjunction with automated or semi-automated medication infusion pumps of the external or implantable type as described in U.S. Pat. No. 4,562,751; 4,678,408; 4,685,903 or 4,573,994, to control delivery of insulin to a diabetic patient.

In the illustrative embodiment shown in Figure 3, the sensor electrodes 10 may be used in a variety of sensing applications and may be configured in a variety of ways. For example, the sensor electrodes 10 may be used in physiological parameter sensing applications in which some type of biomolecule is used as a catalytic agent. For example, the sensor electrodes 10 may be used in a glucose and oxygen sensor having a cellobiose dehydrogenase enzyme catalyzing a reaction with the sensor electrodes 20. The sensor electrodes 10, along with a biomolecule or some other catalytic agent, may be placed in a human body in a vascular or non-vascular environment. For example, the sensor electrodes 20 and biomolecule may be placed in a vein and be subjected to a blood stream, or may be placed in a subcutaneous or peritoneal region of the human body.

In the embodiment shown in Figure 3, the monitor of sensor signals 200 may also be referred to as a sensor electronics device 200. The monitor 200 may include a power source, a sensor interface, processing electronics (i.e. a processor), and data formatting electronics. The monitor 200 may be coupled to the sensor set 10 by a cable 102 through a connector that is electrically coupled to the connector block 28 of the connection portion 24. In an alternative embodiment, the cable may be omitted. In this embodiment, the monitor 200 may include an appropriate connector for direct connection to the connection portion 104 of the sensor set 10. The sensor set 10 may be modified to have the connector portion 104 positioned at a different location, e.g., on top of the sensor set to facilitate placement of the monitor 200 over the sensor set.

While the analyte sensor and sensor systems disclosed herein are typically designed to be implantable within the body of a mammal, the embodiments disclosed herein are not limited to any particular environment and can instead be used in a wide variety of contexts, for example for the analysis of most in vivo and in vitro liquid samples including biological fluids such as interstitial fluids, whole-blood, lymph, plasma, serum, saliva, urine, stool, perspiration, mucus, tears, cerebrospinal fluid, nasal secretion, cervical or vaginal secretion, semen, pleural fluid, amniotic fluid, peritoneal fluid, middle ear fluid, joint fluid, gastric aspirate or the like. In addition, solid or desiccated samples may be dissolved in an appropriate solvent to provide a liquid mixture suitable for analysis.

It is to be understood that this invention is not limited to the particular embodiments described, as such may, of course, vary. It is also to be understood that the terminology used herein is for the purpose of describing particular embodiments only, and is not intended to be limiting, since the scope of the present invention will be limited only by the appended claims. In the description of the preferred embodiment, reference is made to the accompanying drawings which form a part hereof, and in which is shown by way of illustration a specific embodiment in which the disclosure may be practiced. It is to be understood that other embodiments may be utilized and structural changes may be made without departing from the scope of the present invention.

## Claims

1. An amperometric glucose sensor system (100) comprising:
a counter electrode and a reference electrode;
a first working electrode;
an analyte sensing layer (110) disposed over the first working electrode, wherein the analyte sensing layer comprises cellobiose dehydrogenase; and
an analyte modulating layer (112) disposed over the analyte sensing layer,
wherein the analyte modulating layer is configured to modulate the diffusion of glucose as it migrates from an in vivo environment to the analyte sensing layer,
**characterised in that** the analyte modulating layer comprises cellulose acetate in amounts from about 3 wt/% to about 10 wt./%.

2. The amperometric glucose sensor system of claim 1, further comprising a processor, wherein the processor performs the steps of:
assessing electrochemical signal data obtained from the first working electrode; and
computing a glucose concentration based upon the electrochemical signal data obtained from the first working electrode.

3. The amperometric glucose sensor system of claim 2, wherein glucose is sensed by application of a voltage between 0 and 200 millivolts to the working electrode, preferably a voltage less than 100mV, more preferably less than 75mV, even more preferably less than 50mV, and even more preferably less than 40mV or in the range 40mV to 100mV.

4. The amperometric glucose sensor system of any of claims 1 to 3, wherein the working electrode comprises gold or a carbon paste electrode, optionally with an electro-conductive carbon black surface for example KETJENBLACK.

5. The amperometric glucose sensor system of any of claims 1 to 4, wherein the analyte sensing layer comprises a cellobiose dehydrogenase polypeptide preferably in amounts from about 10 mg/mL to about 15 mg/mL.

6. The amperometric glucose sensor system of any of claims 1 to 5, wherein the first working electrode surface comprises ethylene glycol diglycidyl ether (EGDGE) in operable contact with the analyte sensing layer.

7. The amperometric glucose sensor system of any of claims 1 to 6, further comprising:
one or more additional layers disposed over the analyte modulating layer selected from:
a layer comprising poly-l-lysine polymers having molecular weights between 30 KDa and 300KDa; and/or
a layer comprising a polyelectrolyte cationic material.

8. A method of making an electrochemical glucose sensor (100) comprising:
providing a base layer (102);
forming a counter electrode and a reference electrode on the base layer;
forming a conductive layer over the base layer, wherein the conductive layer includes a working electrode;
forming glucose sensing layer (110) over the conductive layer, wherein the glucose sensing layer is selected to include a cellobiose dehydrogenase composition that can alter the electrical current at the working electrode in the conductive layer in the presence of glucose; and
forming an analyte modulating layer (112) comprising cellulose acetate over the glucose sensing layer;
so that the electrochemical analyte sensor is made,
wherein the analyte modulating layer is configured to modulate the diffusion of glucose as it migrates from an in vivo environment to the analyte sensing layer, **characterised in that** the analyte modulating layer comprises cellulose acetate in amounts from about 3 wt/% to about 10 wt./%.

9. The method of claim 8, wherein the glucose sensing layer comprises a cellobiose dehydrogenase polypeptide in amounts from about 5 mg/mL to about 20 mg/mL, and preferably about 10 mg/mL to about 15 mg/mL.

10. The method of any of claims 8 to 9, further comprising disposing an ethylene glycol diglycidyl ether (EGDGE) composition on the working electrode.

11. The method of any of claims 8 to 10, further comprising disposing an electro-conductive carbon black composition, for example KETJENBLACK on the working electrode.

12. The method of any of claims 8 to 11, further comprising:
disposing one or more additional layers over the analyte modulating layer selected from:
a layer comprising poly-l-lysine polymers having molecular weights between 30 KDa and 300KDa;
a layer comprising an albumin;
a layer comprising an adhesion promoting agent; or
a layer comprising a polyelectrolyte cationic layer.

13. The method of any of claims 8 to 12 wherein the working electrode is of gold or carbon.

## Patentansprüche

1. Amperometrisches Glucosesensorsystem (100), umfassend:
eine Gegenelektrode und eine Referenzelektrode;
eine erste Arbeitselektrode;
eine Analyterfassungsschicht (110), die über der ersten Arbeitselektrode angeordnet ist, wobei die Analyterfassungsschicht Cellobiosedehydrogenase umfasst; und
eine Analytmodulationsschicht (112), die über der Analyterfassungsschicht angeordnet ist,
wobei die Analytmodulationsschicht konfiguriert ist, um die Diffusion von Glucose zu modulieren, während sie von einer In-vivo-Umgebung zu der Analytmodulationsschicht wandert, **dadurch gekennzeichnet, dass** die Analytmodulationsschicht Celluloseacetat in Mengen von etwa 3 Gew.-% bis etwa 10 Gew.-% umfasst.

2. Amperometrisches Glucosesensorsystem nach Anspruch 1, ferner umfassend einen Prozessor, wobei der Prozessor die Schritte durchführt:
Auswerten von elektrochemischen Signaldaten, die von der ersten Arbeitselektrode erhalten werden; und
Berechnen einer Glucosekonzentration basierend auf den elektrochemischen Signaldaten, die von der ersten Arbeitselektrode erhalten werden.

3. Amperometrisches Glucosesensorsystem nach Anspruch 2, wobei Glucose durch ein Anlegen einer Spannung zwischen 0 und 200 Millivolt an die Arbeitselektrode erfasst wird, vorzugsweise einer Spannung von weniger als 100 mV, mehr bevorzugt weniger als 75 mV, noch mehr bevorzugt weniger als 50 mV und noch mehr bevorzugt weniger als 40 mV oder in dem Bereich von 40 mV bis 100 mV.

4. Amperometrisches Glucosesensorsystem nach einem der Ansprüche 1 bis 3, wobei die Arbeitselektrode Gold oder eine Kohlenstoffpastenelektrode umfasst, gegebenenfalls mit einer elektrisch leitfähigen Rußoberfläche, zum Beispiel KETJENBLACK.

5. Amperometrisches Glucosesensorsystem nach einem der Ansprüche 1 bis 4, wobei die Analyterfassungsschicht ein Cellobiosedehydrogenase-Polypeptid vorzugsweise in Mengen von etwa 10 mg/ml bis etwa 15 mg/ml, umfasst.

6. Amperometrisches Glucosesensorsystem nach einem der Ansprüche 1 bis 5, wobei die erste Arbeitselektrodenoberfläche Ethylenglycoldiglycidylether (EGDGE) in wirksamem Kontakt mit der Analyterfassungsschicht umfasst.

7. Amperometrisches Glucosesensorsystem nach einem der Ansprüche 1 bis 6, ferner umfassend:
eine oder mehrere zusätzliche Schichten, die über der Analytmodulationsschicht angeordnet sind, ausgewählt aus:
einer Schicht, umfassend Poly-L-Lysin-Polymere, die Molekulargewichte zwischen 30 KDa und 300 Kda aufweisen; und/oder
einer Schicht, umfassend ein kationisches Polyelektrolytmaterial.

8. Verfahren zum Herstellen eines elektrochemischen Glucosesensors (100), umfassend:
Bereitstellen einer Grundschicht (102);
Ausbilden einer Gegenelektrode und einer Referenzelektrode auf der Grundschicht;
Ausbilden einer leitfähigen Schicht über der Grundschicht, wobei die leitfähige Schicht eine Arbeitselektrode einschließt;
Ausbilden einer Glucoseerfassungsschicht (110) über der leitfähigen Schicht, wobei die Glucoseerfassungsschicht ausgewählt ist, um eine Cellobiosedehydrogenase-Zusammensetzung einzuschließen, die den elektrischen Strom an der Arbeitselektrode in der leitfähigen Schicht in der Gegenwart von Glucose verändern kann; und
Ausbilden einer Analytmodulationsschicht (112), umfassend Celluloseacetat, über der Glucoseerfassungsschicht;
sodass der elektrochemische Analytsensor hergestellt wird,
wobei die Analytmodulationsschicht konfiguriert ist, um die Diffusion von Glucose zu modulieren, während sie von einer In-vivo-Umgebung zu der Analytmodulationsschicht wandert, **dadurch gekennzeichnet, dass** die Analytmodulationsschicht Celluloseacetat in Mengen von etwa 3 Gew.-% bis etwa 10 Gew.-% umfasst.

9. Verfahren nach Anspruch 8, wobei die Glucoseerfassungsschicht ein Cellobiosedehydrogenase-Polypeptid in Mengen von etwa 5 mg/ml bis etwa 20 mg/ml und vorzugsweise etwa 10 mg/ml bis etwa 15 mg/ml umfasst.

10. Verfahren nach einem der Ansprüche 8 bis 9, ferner umfassend das Anordnen einer Ethylenglycoldiglycidylether-Zusammensetzung (EGDGE-Zusammensetzung) auf der Arbeitselektrode.

11. Verfahren nach einem der Ansprüche 8 bis 10, ferner umfassend das Anordnen einer elektrisch leitfähigen Rußzusammensetzung, zum Beispiel KETJENBLACK, auf der Arbeitselektrode.

12. Verfahren nach einem der Ansprüche 8 bis 11, ferner umfassend:
Anordnen einer oder mehrerer zusätzlicher Schichten über der Analytmodulationsschicht, ausgewählt aus:
einer Schicht, umfassend Poly-L-Lysin-Polymere, die Molekulargewichte zwischen 30 KDa und 300 Kda aufweisen;
einer Schicht, umfassend ein Albumin;
einer Schicht, umfassend einen Haftvermittler; oder
einer Schicht, umfassend eine kationische Polyelektrolytschicht.

13. Verfahren nach einem der Ansprüche 8 bis 12, wobei die Arbeitselektrode aus Gold oder Kohlenstoff besteht.

## Revendications

1. Système de capteur de glucose ampérométrique (100) comprenant :
une contre-électrode et une électrode de référence ;
une première électrode de travail ;
une couche de détection d'analyte (110) disposée sur la première électrode de travail, dans lequel la couche de détection d'analyte comprend de la cellobiose déshydrogénase ; et
une couche de modulation d'analyte (112) disposée sur la couche de détection d'analyte,
dans lequel la couche de modulation d'analyte est conçue pour moduler la diffusion du glucose lorsqu'il migre d'un environnement in vivo vers la couche de détection d'analyte, **caractérisé en ce que** la couche de modulation d'analyte comprend de l'acétate de cellulose en des quantités allant d'environ 3 % en poids à environ 10 % en poids.

2. Système de capteur de glucose ampérométrique selon la revendication 1, comprenant en outre un processeur, dans lequel le processeur réalise les étapes consistant à :
évaluer des données de signal électrochimique obtenues à partir de la première électrode de travail ; et
calculer une concentration en glucose en fonction des données de signal électrochimique obtenues à partir de la première électrode de travail.

3. Système de capteur de glucose ampérométrique selon la revendication 2, dans lequel le glucose est détecté par l'application d'une tension comprise entre 0 et 200 millivolts à l'électrode de travail, de préférence une tension inférieure à 100 mV, plus préférablement inférieure à 75 mV, encore plus préférablement inférieure à 50 mV, et encore plus préférablement inférieure à 40 mV ou dans la plage de 40 mV à 100 mV.

4. Système de capteur de glucose ampérométrique selon l'une quelconque des revendications 1 à 3, dans lequel l'électrode de travail comprend de l'or ou une électrode en pâte de carbone, éventuellement avec une surface en noir de carbone électroconductrice, par exemple KETJENBLACK.

5. Système de capteur de glucose ampérométrique selon l'une quelconque des revendications 1 à 4, dans lequel la couche de détection d'analyte comprend un polypeptide de cellobiose déshydrogénase, de préférence en des quantités allant d'environ 10 mg/ml à environ 15 mg/ml.

6. Système de capteur de glucose ampérométrique selon l'une quelconque des revendications 1 à 5, dans lequel la surface de première électrode de travail comprend de l'éther diglycidylique d'éthylène glycol (EGDGE) en contact opérationnel avec la couche de détection d'analyte.

7. Système de capteur de glucose ampérométrique selon l'une quelconque des revendications 1 à 6, comprenant en outre :
une ou plusieurs couches supplémentaires disposées sur la couche de modulation d'analyte choisie parmi :
une couche comprenant des polymères de poly-L-lysine dont le poids moléculaire est compris entre 30 KDa et 300 KDa ; et/ou
une couche comprenant un matériau cationique de polyélectrolyte.

8. Procédé de fabrication d'un capteur de glucose électrochimique (100) comprenant :
la fourniture d'une couche de base (102) ;
la formation d'une contre-électrode et d'une électrode de référence sur la couche de base ;
la formation d'une couche conductrice sur la couche de base, dans lequel la couche conductrice comporte une électrode de travail ;
la formation d'une couche de détection de glucose (110) sur la couche conductrice, dans lequel la couche de détection de glucose est choisie pour comporter une composition de cellobiose déshydrogénase qui peut modifier le courant électrique au niveau de l'électrode de travail dans la couche conductrice en présence de glucose ; et
la formation d'une couche de modulation d'analyte (112) comprenant de l'acétate de cellulose sur la couche de détection du glucose ;
de sorte que le capteur d'analyte électrochimique est fabriqué,
dans lequel la couche de modulation d'analyte est conçue pour moduler la diffusion du glucose lorsqu'il migre d'un environnement in vivo vers la couche de détection d'analyte, **caractérisé en ce que** la couche de modulation d'analyte comprend de l'acétate de cellulose en des quantités allant d'environ 3 % en poids à environ 10 % en poids.

9. Procédé selon la revendication 8, dans lequel la couche de détection de glucose comprend un polypeptide de cellobiose déshydrogénase en quantités allant d'environ 5 mg/ml à environ 20 mg/ml, et de préférence d'environ 10 mg/ml à environ 15 mg/ml.

10. Procédé selon l'une quelconque des revendications 8 à 9, comprenant en outre la disposition d'une composition d'éther diglycidylique d'éthylène glycol (EGDGE) sur l'électrode de travail.

11. Procédé selon l'une quelconque des revendications 8 à 10, comprenant en outre la disposition d'une composition de noir de carbone électroconductrice, par exemple KETJENBLACK, sur l'électrode de travail.

12. Procédé selon l'une quelconque des revendications 8 à 11, comprenant en outre :
la disposition d'une ou plusieurs couches supplémentaires sur la couche de modulation d'analyte choisie parmi :
une couche comprenant des polymères de poly-L-lysine dont le poids moléculaire est compris entre 30 KDa et 300 KDa ;
une couche comprenant une albumine ;
une couche comprenant un agent favorisant l'adhérence ; ou
une couche comprenant une couche cationique de polyélectrolyte.

13. Procédé selon l'une quelconque des revendications 8 à 12, dans lequel l'électrode de travail est en or ou en carbone.
